# EUROPEAN PATENT APPLICATION

(11) **EP 4 300 085 A1**
(43) Date of publication of application: **03.01.2024**
(21) Application number: 22759781.2
(22) Date of filing: 25.02.2022
(51) Int. Cl.: G01N 23/18

(54) **IMAGE DISPLAY DEVICE, IMAGE DISPLAY METHOD AND PROGRAM**

(30) Priority: 26.02.2021 JP 2021031134
(71) Applicant: FUJIFILM Corporation, Tokyo 106-8620 (JP)
(72) Inventor: IKEDA, Haruka, Tokyo 106-8620 (JP)
(74) Representative: Meissner Bolte Partnerschaft mbB
(86) International application number: PCT/JP2022/007787
(87) International publication number: WO 2022/181747

(57) **Abstract**

Provided are an image display device, an image display method, and a program which are capable of improving the accuracy of determination by comprehensively considering interpretation information by a plurality of determiners. A first inspection target image in which an inspection object is imaged is displayed on a display, a marker in at least one region with which the interpretation information of the first inspection target image is associated is displayed, a second inspection target image subjected to at least one of enlargement processing or highlight processing for a region including at least a part of a region of the selected marker is displayed on the display, at least one of first information, which is document information of the interpretation information associated with the region of the marker selected by a user, or second information, which is statistical information obtained by statistically processing non-document information of the interpretation information, is displayed on the display.

## Description

### BACKGROUND OF THE INVENTION

### 1. Field of the Invention

The present invention relates to an image display device, an image display method, and a program, and more particularly relates to a technique for determining a defect of an inspection object from an image of the inspection object.

### 2. Description of the Related Art

A device that, in a case of interpretation for a medical image, allows two users to input a position of a lesion in an image, extracts the position where determination results regarding the presence or absence of the lesion are different, and highlights and displays a region determined as a user tends to make different determination from another user based on the difference is described (JP2012-223506A).

### SUMMARY OF THE INVENTION

In non-destructive inspection and various inspection tasks in a field of social infrastructure, it is necessary to set a determination criterion according to individual cases. Therefore, determination accuracy can be improved by comparing the determination results by a plurality of interpreters. In addition, in a case in which a large number of inspection targets of the same type are determined, such as standard products, it may be useful to accumulate the results of determinations in the past in some cases.

However, in the technique described in JP2012-223506A, the determination accuracy cannot be improved by using the determination result.

The present invention has been made in view of such circumstances, and an object of the present invention is to provide an image display device, an image display method, and a program which are capable of improving the accuracy of determination by comprehensively considering interpretation information by a plurality of determiners.

One aspect of an image display device for achieving the above object is an image display device comprising at least one processor, and at least one memory that stores an instruction to be executed by the at least one processor, in which the at least one processor acquires a first inspection target image in which a first inspection object is imaged and in which interpretation information by a plurality of determiners is associated with an arbitrary region, displays the first inspection target image on a display and displays a marker in at least one region with which the interpretation information of the first inspection target image is associated, receives a selection instruction of the marker by a user, displays a second inspection target image subjected to at least one of enlargement processing or highlight processing for a region including at least a part of a region of the marker for which the selection instruction is received in the first inspection target image, on the display, and further displays at least one of first information, which is document information of the interpretation information associated with the region of the marker for which the selection instruction is received, or second information, which is statistical information obtained by statistically processing non-document information of the interpretation information, on the display. According to the aspect, the user can consider the interpretation information by the plurality of determiners without omission.

It is preferable that the document information includes a plurality of comments input by the plurality of determiners, respectively, and the at least one processor displays a list of the plurality of comments.

It is preferable that the non-document information includes determination results each classified by the plurality of determiners and including at least one of presence or absence, a type, or a severity of a defect in the region of the marker, and the statistical information obtained by statistically processing the non-document information includes a distribution of the number of persons for each determination result.

It is preferable that the non-document information includes numerical information of a defect determined by each of the plurality of determiners, and the statistical information obtained by statistically processing the non-document information includes at least one of mean or a distribution of the numerical information of the defect.

It is preferable that the numerical information of the defect includes at least one of a size of the defect or a density of the defect.

It is preferable that the first inspection target image includes information of image processing in a case in which a determination, which is a basis of the interpretation information, is made, and the at least one processor receives a selection instruction by the user of at least one of the first information or the second information displayed on the display, and displays the first inspection target image subjected to the image processing in a case in which a determination, which is a basis of the first information or second information for which the selection instruction is received, is made, on the display.

It is preferable that the at least one processor displays the first inspection target image subjected to the image processing together with the second inspection target image on the display.

It is preferable that the at least one processor displays the first inspection target image subjected to the image processing on the display in place of the second inspection target image.

It is preferable that the image processing includes at least one of reproduction of an enlargement ratio of an image, reproduction of a display position of the image, or a parameter of an appearance of the image, and the parameter of the appearance of the image includes at least one of lightness and darkness of the image, sharpness of the image, contrast of the image, or gradation of the image.

It is preferable that the at least one processor receives at least one of correction or approval of the interpretation information associated with the region, and superimposes and displays a mark indicating a final determination on the region in which the at least one of correction or approval has been received.

It is preferable that the at least one processor receives an input of a region of the first inspection target image that needs to be redetermined, and superimposes and displays a mark indicating remand on the input region.

It is preferable that the at least one processor acquires determined interpretation information of a determined image in which a second inspection object different from the first inspection object is imaged, and in which the determined interpretation information by the plurality of determiners is associated with an arbitrary region, and displays at least one of third information, which is document information of the determined interpretation information related to the interpretation information associated with the region of the marker for which the selection instruction is received, or fourth information, which is statistical information obtained by statistically processing non-document information of the determined interpretation information on the display.

It is preferable that the second inspection object is the same standard product as the first inspection object.

It is preferable that the displayed document information of the determined interpretation information includes at least one of the same text as the document information of the interpretation information associated with the region of the marker for which the selection instruction is received, or text indicating a determination result for the same defect.

It is preferable that the at least one processor calculates a distribution of determination results of the plurality of determiners for the first inspection target image, and in a case in which a variation in the determination result with respect to the first inspection target image is larger than that of a first criterion, displays that the determination is varied with respect to the determination results of the plurality of determiners for the first inspection target image on the display.

It is preferable that the at least one processor calculates a distribution of determination results of the plurality of determiners for the first inspection target image, and in a case in which a determination result of a specific determiner with respect to the first inspection target image is an outlier, displays that there is a possibility that the determination result of the specific determiner is the outlier on the display.

It is preferable that the at least one processor performs the statistical processing on the non-document information of the interpretation information by weighting the determination result of each of the plurality of determiners based on a determination record of each of the determiners.

One aspect of an image display method for achieving the above object is an image display method comprising: an inspection target image acquisition step of acquiring a first inspection target image in which a first inspection object is imaged and in which interpretation information by a plurality of determiners is associated with an arbitrary region; a first image display step of displaying the first inspection target image on a display and displaying a marker in at least one region with which the interpretation information of the first inspection target image is associated; a reception step of receiving a selection instruction of the marker by a user; a second image display step of displaying a second inspection target image subjected to at least one of enlargement processing or highlight processing for a region including at least a part of a region of the marker for which the selection instruction is received in the first inspection target image, on the display; and an interpretation information display step of further displaying at least one of first information, which is document information of the interpretation information associated with the region of the marker for which the selection instruction is received, or second information, which is statistical information obtained by statistically processing non-document information of the interpretation information, on the display. According to the aspect, the user can consider the interpretation information by the plurality of determiners without omission.

One aspect of a program for achieving the above object is a program for causing a computer to execute the image display method. A computer-readable non-transitory storage medium on which the program is recorded may also be included in the aspect.

According to the present invention, it is capable of improving the accuracy of determination by comprehensively considering interpretation information by a plurality of determiners.

### BRIEF DESCRIPTION OF THE DRAWINGS

Fig. 1 is a block diagram illustrating a defect inspection system.
Fig. 2 is a block diagram illustrating an example of an imaging system.
Fig. 3 is a flowchart illustrating steps of an image display method.
Fig. 4 is a diagram illustrating an example of a screen of a display on which an inspection target image is displayed.
Fig. 5 is a diagram illustrating an example of marker selection by a user.
Fig. 6 is a diagram illustrating an example of a screen of a display on which an enlarged inspection target image is displayed.
Fig. 7 is a diagram illustrating an example of a screen of a display on which a highlighted inspection target image is displayed.
Fig. 8 is a diagram illustrating an example of a screen of a display in a case in which document information is selected.
Fig. 9 is a diagram illustrating an example of a screen of a display in a case in which document information is selected.
Fig. 10 is a diagram illustrating an example of a screen of a display on which a final determination mark is superimposed and displayed.
Fig. 11 is a diagram illustrating an example of a screen of a display on which a mark indicating remand is superimposed and displayed.
Fig. 12 is a diagram illustrating an example of statistical information displayed on a screen of a display.

### DESCRIPTION OF THE PREFERRED EMBODIMENTS

Hereinafter, a preferred embodiment of the present invention will be described in accordance with the accompanying drawings.

### [Image display device]

An image display device according to the present embodiment is used in a defect inspection system of non-destructive inspection. The defect inspection system is a system in which a user determines a defect of an inspection object from an image in which an industrial product, which is the inspection object, is imaged. Here, an example will be described in which the user determines the defect in consideration of interpretation information of a plurality of determiners associated with the image of the inspection object. The user is an interpreter, and may be any person of the plurality of determiners, or may be a person other than the determiners. Here, a case in which an inspection target image of the inspection object is a radiation image of an industrial product will be described, but the inspection target image may be other objects and other interpretation target images. In addition, the industrial product which is the inspection object is not particularly limited in material such as metal or resin, and may be before or after processing, or may be a part or an assembly.

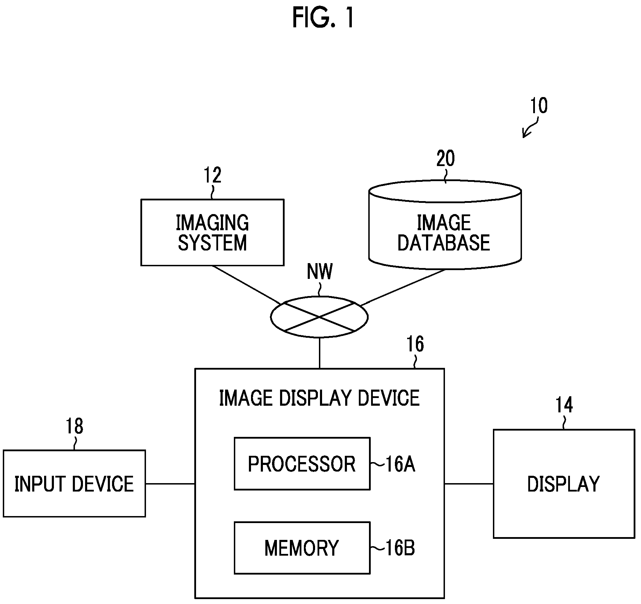

Fig. 1 is a block diagram illustrating a defect inspection system 10 according to the present embodiment. As illustrated in Fig. 1, the defect inspection system 10 comprises an imaging system 12, a display 14, an image display device 16, an input device 18, and an image database 20.

The imaging system 12 is a system that captures an image of an inspection object OBJ, which is a first inspection object. Details regarding the imaging system 12 will be described later.

The display 14 is a display device for allowing the user to visually recognize an inspection target image or the like in which the inspection object OBJ is imaged. The display 14 displays a screen necessary for an operation of the input device 18, and functions as a part for implementing a graphical user interface (GUI).

The image display device 16 is a control device for displaying an image of the inspection object OBJ imaged by the imaging system 12 on the screen of the display 14. The image display device 16 comprises a processor 16A and a memory 16B.

The processor 16A executes an instruction stored in the memory 16B. A plurality of processors 16A may be comprised.

A hardware structure of the processor 16A is various processors as described below. The various processors include a central processing unit (CPU) that is a general-purpose processor actioning as various functional units by executing software (program), a graphics processing unit (GPU) that is a processor specially designed for image processing, a programmable logic device (PLD) such as a field programmable gate array (FPGA) that is a processor having a circuit configuration changeable after manufacturing, and a dedicated electric circuit or the like such as an application specific integrated circuit (ASIC) that is a processor having a circuit configuration dedicatedly designed to execute a specific type of processing.

One processing unit may be configured by one processor among these various processors, or may be configured by two or more same or different kinds of processors (for example, a combination of a plurality of FPGAs, a combination of the CPU and the FPGA, or a combination of the CPU and GPU). In addition, a plurality of functional units may be formed of one processor. As an example of configuring the plurality of functional units with one processor, first, as represented by a computer such as a client or a server, a form of configuring one processor with a combination of one or more CPUs and software and causing the processor to act as the plurality of functional units is present. Second, as represented by a system on chip (SoC) or the like, a form of using a processor that implements the function of the entire system including the plurality of functional units using one integrated circuit (IC) chip is present. Accordingly, various functional units are configured using one or more of the various processors as a hardware structure.

Furthermore, the hardware structure of the various processors is more specifically an electric circuit (circuitry) in which circuit elements such as semiconductor elements are combined.

The memory 16B stores instructions to be executed by the processor 16A. The memory 16B includes a random access memory (RAM) and a read only memory (ROM), which are not illustrated. The processor 16A uses the RAM as a work region, executes software using various programs and parameters including an image display program stored in the ROM, and executes various processing of the image display device 16 using the parameters stored in the ROM or the like.

The input device 18 is an interface that receives an operation input from the user. The input device 18 includes a keyboard for inputting characters and a pointing device for operating a pointer, an icon, or the like displayed on the screen of the display 14. The input device 18 may be a touch-panel display integrated with the display 14.

The image database 20 is composed of a high-capacity storage device. The image database 20 stores a plurality of determined images, each of which is captured in the past, of a plurality of different second inspection objects. In each of the determined images, the interpretation information including determination results of defects of the second inspection object by the plurality of determiners is associated with an arbitrary region, respectively. The second inspection object is an article different from the inspection object OBJ. The second inspection object may be, for example, the same standard product as the inspection object OBJ or an article having a different shape from the inspection object OBJ.

The imaging system 12, the image display device 16, and the image database 20 are communicably connected through a network NW. The network NW is, for example, a local area network (LAN), a wide area network (WAN), or an Internet connection. The imaging system 12, the image display device 16, and the image database 20 may be communicably connected by a universal serial bus (USB) cable or the like, or may be communicably connected by short-range wireless communication using infrared rays or the like. The imaging system 12, the image display device 16, and the image database 20 may exchange data including an image through a storage medium that is detachable and readable, respectively.

### [Imaging system]

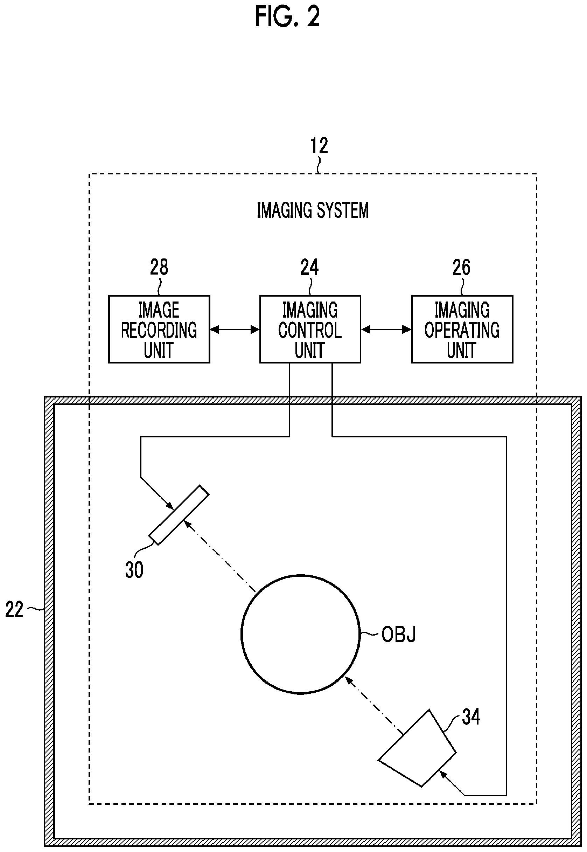

Fig. 2 is a block diagram illustrating an example of an imaging system 12. As illustrated in Fig. 2, the imaging system 12 comprises an imaging room 22, an imaging control unit 24, an imaging operating unit 26, an image recording unit 28, a radiation detector 30, and a radiation source 34.

In the imaging room 22, a partition wall and the entrance/exit between the imaging room 22 and the outside are protected from X-ray by an X-ray protective material (for example, lead, concrete, or the like). In addition to the radiation detector 30 and the radiation source 34, an inspection object OBJ, which is an imaging target, is disposed inside the imaging room 22.

The imaging control unit 24 includes a CPU that controls an operation of each unit of the imaging system 12. The imaging control unit 24 receives an operation input from an imaging technician through the imaging operating unit 26, and transmits a control signal corresponding to the received operation input to each unit of the imaging system 12 to control the operation of each unit.

The imaging operating unit 26 is an input device that receives an operation input from the imaging technician. The imaging technician can perform, through the imaging operating unit 26, an input of information regarding the inspection object OBJ, an input of an instruction to execute imaging to the radiation detector 30 (including settings for imaging conditions such as exposure time, focal length, and stop, imaging angle, and imaging location), an input of an instruction of radiation irradiation to the radiation source 34 (including settings for irradiation start time, irradiation duration, irradiation angle, and irradiation intensity) and an input of an instruction to record the acquired image in the image recording unit 28.

The image recording unit 28 records an image (light-receiving image data) of the inspection object OBJ, which is imaged by the radiation detector 30. The image recording unit 28 records information for specifying the inspection object OBJ in association with the image.

For example, the radiation source 34 is an X-ray source. The radiation source 34 irradiates the inspection object OBJ in the imaging room 22 with radiation in response to an instruction from the imaging control unit 24. In a case in which the inspection object OBJ is irradiated with visible light for imaging, it is not necessary to use the imaging room 22 with protection.

The radiation detector 30 receives the radiation irradiated from the radiation source 34 to the inspection object OBJ and transmitted through the inspection object OBJ, and images the inspection object OBJ in response to an instruction to execute imaging from the imaging control unit 24. The inspection object OBJ is held in the imaging room 22 by a holding member (for example, a manipulator, a mounting table, or a movable mounting table) which is not illustrated, and the distance and the angle of the inspection object OBJ with respect to the radiation detector 30 and the radiation source 34 can be adjusted. The operator can control relative positions of the inspection object OBJ, the radiation detector 30, and the radiation source 34 through the imaging control unit 24, and can image a desired location of the inspection object OBJ.

The radiation source 34 ends the irradiation of the radiation to the inspection object OBJ in synchronization with the end of the execution of the imaging by the radiation detector 30.

In an example illustrated in Fig. 2, the radiation detector 30 is disposed inside the imaging room 22, but the radiation detector 30 may be disposed outside the imaging room 22 as long as it can image the inspection object OBJ in the imaging room 22.

In addition, in the example illustrated in Fig. 2, one radiation detector 30 and one radiation source 34 are provided, but the number of radiation detectors and radiation sources is not limited thereto. For example, a plurality of the radiation detectors and a plurality of the radiation sources may be provided.

### [Image display method]

An image display method using the image display device 16 will be described. The image display method is implemented by the processor 16A executing an image display program stored in the memory 16B. The image display program may be provided by a computer-readable non-transitory storage medium. In this case, the image display device 16 may read the image display program from the non-transitory storage medium and store it in the memory 16B.

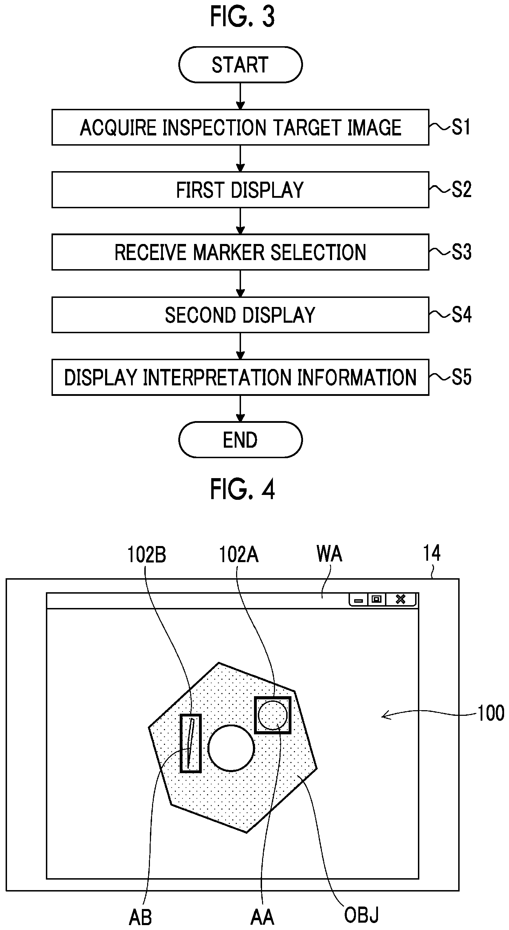

Fig. 3 is a flowchart illustrating steps of the image display method. In the image display method according to the present embodiment, an image in which the inspection object OBJ is imaged and interpretation information thereof are displayed on the screen of the display 14.

The processor 16A of the image display device 16 acquires a first inspection target image in which the inspection object is imaged (Step S 1: an example of an inspection target image acquisition step). Here, the processor 16A acquires an inspection target image 100 in which the inspection object OBJ is imaged from the image database 20 through a network NW. The inspection target image 100 is an image in which the inspection object OBJ is imaged in the imaging system 12, and is an image that has already been interpreted by the plurality of determiners. In the inspection target image 100, the interpretation information by the plurality of the determiners is associated with an arbitrary region in advance.

Next, the processor 16A displays the acquired first inspection target image on the display, and displays a marker in at least one region associated with the interpretation information of the first inspection target image (Step S2: an example of a first image display step).

Fig. 4 is a diagram illustrating an example of the screen of the display 14 on which the inspection target image 100 is displayed. In an example illustrated in Fig. 4, a window WA is displayed on the screen of the display 14, and the inspection target image 100 is displayed in the window WA.

Here, it is assumed that the inspection target image 100 has interpretation information associated with a region AA and a region AB, respectively. In addition, it is assumed that in the inspection target image 100, a marker 102A is given to the region AA associated with the interpretation information and a marker 102B is given to the region AB. In the example illustrated in Fig. 4, the marker 102A and the marker 102B are bounding boxes surrounding the region AA and the region AB, respectively, but the marker 102A and the marker 102B are not limited to bounding boxes and may be in other forms as long as it is recognizably displayed that the interpretation information is associated with the region AA and the region AB.

In the first inspection target image, the marker may not be given in advance to the region associated with the interpretation information. In this case, the processor 16A superimposes and displays the marker on the region associated with the interpretation information. For example, in a case in which the marker is not given to the inspection target image 100, the processor 16A may superimpose and display the marker 102A on the region AA and the marker 102B in the region AB in the inspection target image 100 displayed in the window WA. Accordingly, the processor 16A can perform the same display as in a case in which the marker is given in advance to the first inspection target image.

Returning to the description of Fig. 3, the processor 16A receives a marker selection instruction by the user (Step S3: an example of a reception step). The user uses the input device 18 to select and instruct the marker 102A or the marker 102B of the inspection target image 100 displayed on the screen of the display 14. The processor 16A receives the marker 102A or the marker 102B selected and instructed by the input device 18.

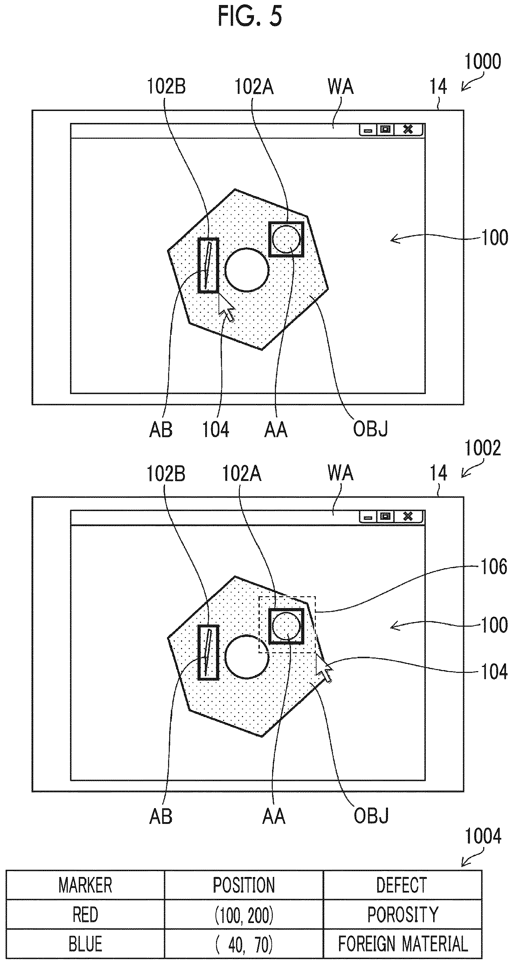

Fig. 5 is a diagram illustrating an example of a marker selection instruction by the user. 1000 and 1002 illustrated in Fig. 5 illustrate examples of using a pointing device (not illustrated) to select and instruct the markers, respectively. In the example illustrated in 1000, the user selects and instructs the marker 102B by placing a cursor 104 on a desired position of the marker 102B and performing a clicks operation. In addition, in the example illustrated in 1002, the user selects and instructs the marker 102A by designating a range 106 surrounding the marker 102A by a drag operation.

In addition, 1004 illustrated in Fig. 5 is a list of marker information displayed on the screen of the display 14. The list of the marker information may be displayed together with the inspection target image 100 by being displayed in a different window from the window WA.

As illustrated in 1004, the marker information includes information of a marker color, a marker position, and a type of a defect in a region associated with the marker. Here, by displaying the marker 102A in red and the marker 102B in blue, a plurality of markers are identified by color, but the markers may be distinguished by information other than the color. The marker information may include a representative determination result of the defect in the region associated with the marker. The marker information may include enough information to identify each marker.

The user selects the desired marker information from the list of the marker information using the input device 18, so that the user can select and instruct the marker of the information.

The marker information and the cursor 104 may be linked with each other. For example, only the information of the marker corresponding to the marker closest to the position of the cursor 104 on the image may be displayed, or the information of the marker corresponding to the marker closest to the position of the cursor 104 on the image in the list of the information of the marker may be highlighted and displayed.

Here, it is assumed that the processor 16A receives the selection instruction of the marker 102A by the user.

Returning to the description of Fig. 3 again, in a case of receiving the marker selection instruction by the user, the processor 16A causes the display to display a second inspection target image subjected to at least one of enlargement processing or highlight processing for a region including at least a part of the selected marker region of the first inspection target image (Step S4: an example of a second image display step).

Furthermore, the processor 16A causes the display to display at least one of first information, which is document information of interpretation information associated with the region of the selected marker, or second information, which is statistical information obtained by performing statistical processing for non-document information of the interpretation information (Step S5: an example of an interpretation information display step). The document information and non-document information will be described later. This is the end of processing of the present flowchart.

Since the selection instruction of the marker 102A by the user is received in Step S3, the processor 16A generates the second inspection target image subjected to at least one of the enlargement processing or the highlight processing for a region including at least a part of the region AA of the inspection target image 100. It is not necessary that the region AA and the region where at least one of the enlargement processing or the highlight processing is performed coincide with each other, and the processing may be performed including a region around the region AA, or may be performed on a partial region of the region AA. Here, the processor 16A generates an enlarged inspection target image 110 in which a part of the region AAis enlarged at a predetermined enlargement ratio as the second inspection target image, and displays the generated enlarged inspection target image 110 on the screen of the display 14.

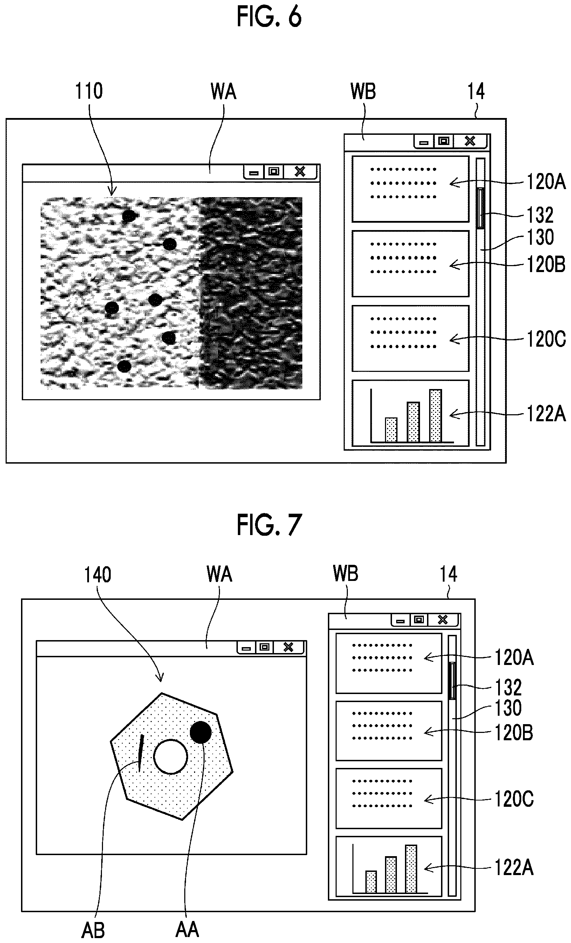

Fig. 6 is a diagram illustrating an example of the screen of the display 14 on which the enlarged inspection target image 110 is displayed. In an example illustrated in Fig. 6, the enlarged inspection target image 110 is displayed in the window WA. In the enlarged inspection target image 110 displayed in the window WA of Fig. 6, the region AA is enlarged at a predetermined enlargement ratio with respect to the inspection target image 100 displayed in the window WA of Fig. 4. In this way, by shifting from the display of the entire inspection object OBJ to the enlarged display of the region AA, the region AA is displayed with a higher resolution and in detail, so that the user can visually recognize the region AA in detail.

In addition, since the selection instruction of the marker 102A by the user is received in Step S3, the processor 16A reads out the interpretation information associated with the region AA from the image database 20. The interpretation information may be included in the inspection target image 100 as metadata of the inspection target image 100.

The interpretation information includes the document information. The document information includes a plurality of comments each input by the plurality of determiners. The input comments may include a basis for determination, for example, "because a large number of defects are confirmed at the time of enlargement". Here, it is assumed that the region AA is associated with at least document information 120A of a determiner A who has determined the inspection target image 100, document information 120B of a determiner B, and document information 120C of a determiner C as the document information of the interpretation information. Therefore, the processor 16A causes the display 14 to display a list of at least the document information 120A, 120B, and 120C as the first information. In the example illustrated in Fig. 6, the document information 120A, 120B, and 120C are displayed in a window WB.

In addition, the interpretation information includes the non-document information. The non-document information includes determination results of defects determined by the plurality of determiners or numerical information of the defects. Here, it is assumed that the non-document information of the interpretation information is associated with the region AA. Therefore, the processor 16A performs statistical processing for the non-document information and displays the statistically processed statistical information 122A on the screen of the display 14 as the second information. In the example illustrated in Fig. 6, the statistical information 122A is displayed in the window WB.

In this way, by displaying the document information 120A, 120B, and 120C and the statistical information 122A on the screen of the display 14 in addition to the enlarged inspection target image 110, the user can confirm the information of the region AA without omission and can consider the interpretation information by the plurality of determiners without omission.

In addition, the processor 16A displays a scroll bar 130 and a slider 132 in the window WB. The user can use the input device 18 to move the slider 132 in an up-down direction along the scroll bar 130, and the processor 16A displays the first information and the second information disposed in the up-down direction in the window WB on the screen of the display 14 according to a position of the slider 132. In this way, even in a case in which all of the first information and the second information cannot be displayed on the display 14 at one time, the first information and the second information can be displayed on the screen of the display 14 without omission. Therefore, the user can confirm and consider the interpretation information by the plurality of determiners without omission.

Here, the processor 16A displays the enlarged inspection target image 110 in place of the inspection target image 100, but may display the enlarged inspection target image 110 in a window different from the window WA in which the inspection target image 100 is displayed, and simultaneously display both the inspection target image 100 and the enlarged inspection target image 110.

### [Highlight processing]

In an example illustrated in Fig. 6, the processor 16A displays the enlarged inspection target image 110 in which a part of the region AA is enlarged as the second inspection target image on the screen of the display 14, but the highlighted inspection target image subjected to the highlight processing of the region AA as the second inspection target image may be displayed on the screen of the display 14.

Fig. 7 is a diagram illustrating an example of the screen of the display 14 on which a highlighted inspection target image 140 subjected to the highlight processing for the region AA is displayed. In the example illustrated in Fig. 7, the highlighted inspection target image 140 is displayed in the window WA.

Here, the processor 16A generates the highlighted inspection target image 140 by changing the entire gradation of the inspection target image 100. The highlight processing of the region AA is not limited to the gradation change processing, and may be sharpness change processing, brightness change processing, or the like. In addition, the processor 16A may perform highlight processing for at least a part of the region AA. The marker 102A and the marker 102B may be displayed on the highlighted inspection target image 140.

In addition, the processor 16A may generate an enlargement-highlighted inspection target image subjected to both the enlargement processing and the highlight processing of the region AA as the second inspection target image and display the image on the screen of the display 14.

### [Non-document information and statistical information]

Statistic is "to investigate the distribution of individual elements in a group and to quantitatively and uniformly clarify trends and characteristics of the group. In addition, the numerical values obtained as a result.". Statistical information obtained by statistically processing non-document information includes what is generally referred to as "statistics".

The non-document information is determination results of defects, each classified by the plurality of determiners, and includes, for example, at least one of the presence or absence, type, or severity of the defect in the region of the marker. In addition, the statistical information of the determination result of the defect may be the distribution of the number of persons for each determination result. The distribution of the number of persons may be illustrated using any of a histogram, a selection rate for each determination, a circle graph, or a bar graph.

In addition, the non-document information may be numerical information of defects determined by each of the plurality of determiners, or may be a confidence level of the determination and a measure of the severity. The numerical information of the defect may be a size (area) of the defect, the density as a group of defects that occur in close proximity rather than the simple size of individual defects, or the position coordinates (position of centroid, position of bounding box) of the defects. In addition, the numerical information of the defect may be a marker given to the region in advance or a diagonal length of a circumscribed rectangle which is a newly given bounding box. In addition, the diagonal length of the circumscribed rectangle in consideration of rotation or a graphic parameter in a case in which elliptical fitting is performed may be used. The diagonal length of the circumscribed rectangle in consideration of the rotation makes it possible to distinguish between an elongated shape of defect and a round shape of defect.

The statistical information including the numerical information of the defect, the confidence level of the determination, and the measure of severity may be at least one of their mean, variance, deviation, maximum and minimum, or histogram. The mean may be any of simple mean, weighted mean, or mean excluding outliers. For the weighted mean, the weight for each determiner may be used.

### [Display of image in a case in which interpretation information is created]

The processor 16A may display an image in a case in which the interpretation information is created on the screen of the display 14. For example, the inspection target image 100 includes information of image processing in a case in which a determination, which is a basis of the interpretation information associated with the region AA, is made, and the processor 16A receives a selection instruction by the user of at least one of the document information 120A, the document information 120B, the document information 120C, or the statistical information 122A displayed on the screen of the display 14, performs the image processing, in a case in which a determination, which is a basis of the selected information, is made, on the inspection target image 100, and displays the image on the screen of the display 14.

Here, the information of the image processing includes at least one of reproduction of an enlargement ratio (the size to be displayed) of the image, reproduction of a display position of the image, or a parameter of an appearance of the image. The parameter of the appearance of the image includes at least one of lightness and darkness of the image, sharpness of the image, contrast of the image, or gradation of the image.

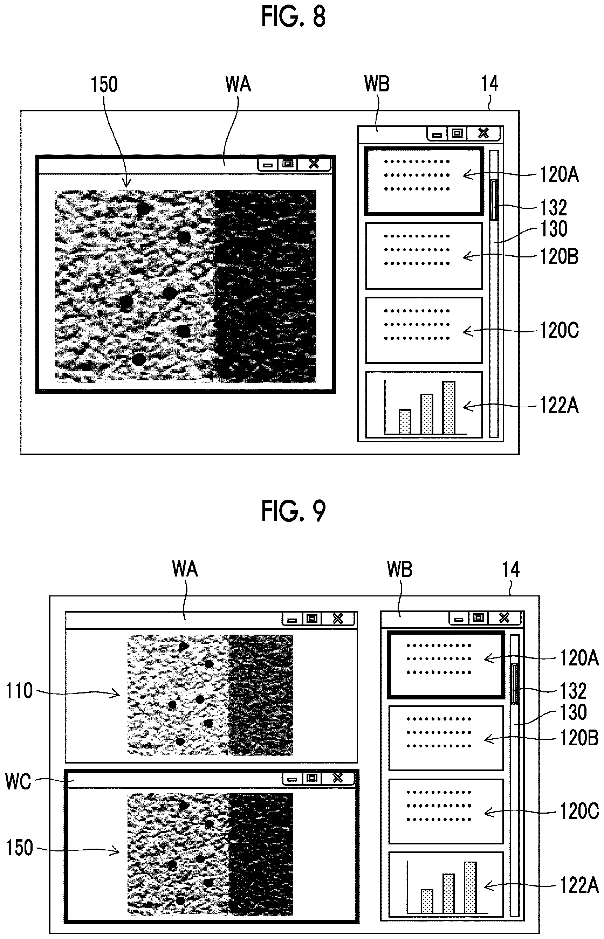

Fig. 8 is a diagram illustrating an example of the screen of the display 14 in a case in which the user uses the input device 18 and selects the document information 120A in a state illustrated in Fig. 6. In a case in which the document information 120A is selected, the processor 16A acquires image processing information from the image database 20 in a case in which the determiner A creates the document information 120A from the inspection target image 100, applies the acquired image processing to the inspection target image 100 to generate a document creation image 150, and displays the image on the screen of the display 14. The inspection target image 100 may have information of the image processing in a case in which the determiner A creates the document information 120A as metadata of the inspection target image 100.

As illustrated in Fig. 8, the processor 16A displays a line of a frame of the selected document information 120A to be thicker than a line of a frame of the other interpretation information. In addition, in place of the enlarged inspection target image 110, the processor 16A displays, on the window WA, the document creation image 150, which is an image of the region AA used in a case in which the determiner A creates the document information 120A. In addition, the processor 16A displays a frame of the window WA in a case of displaying the document creation image 150 to be thicker than a frame of the window WA in a case of displaying the enlarged inspection target image 110. Here, although the line of the frame is displayed thick, it is sufficient to identify that the document information 120A corresponds to the document creation image 150. For example, a display method may be used in which a color of the line of the frame of the document information 120A is displayed in the same color as at least one color of a frame of the window WA or a menu bar.

By performing such screen display, the user can visually recognize images displayed at the time of creating the document information and the non-document information.

The document creation images 150 may be disposed side by side so as to be comparable with the enlarged inspection target image 110. Fig. 9 is a diagram illustrating the display 14 in a case in which the user uses the input device 18 and selects the document information 120A in the state illustrated in Fig. 6, and is a diagram of a case in which the enlarged inspection target image 110 and the document creation image 150 are displayed in a separate window at the same time. In an example illustrated in Fig. 9, the enlarged inspection target image 110 is displayed in the window WA, and the document creation image 150 is displayed in another window WC.

By performing such screen display, the user can visually recognize images displayed at the time of creating the document information and the non-document information, and can compare the enlarged inspection target image 110 with the document creation image 150.

### [Approval and remand]

The interpretation information may be configured to be approvable by the user, or the interpretation information may be configured to be corrected before the approval. For example, the processor 16A receives at least one of correction or approval of at least one information of the document information 120A, the document information 120B, the document information 120C, or the statistical information 122A, which are interpretation information associated with the region AA, and superimposes and displays a mark indicating the final determination on the region AA in which at least one of the correction or the approval has been received. The user can correct and approve the document information 120A or the like by using the input device 18.

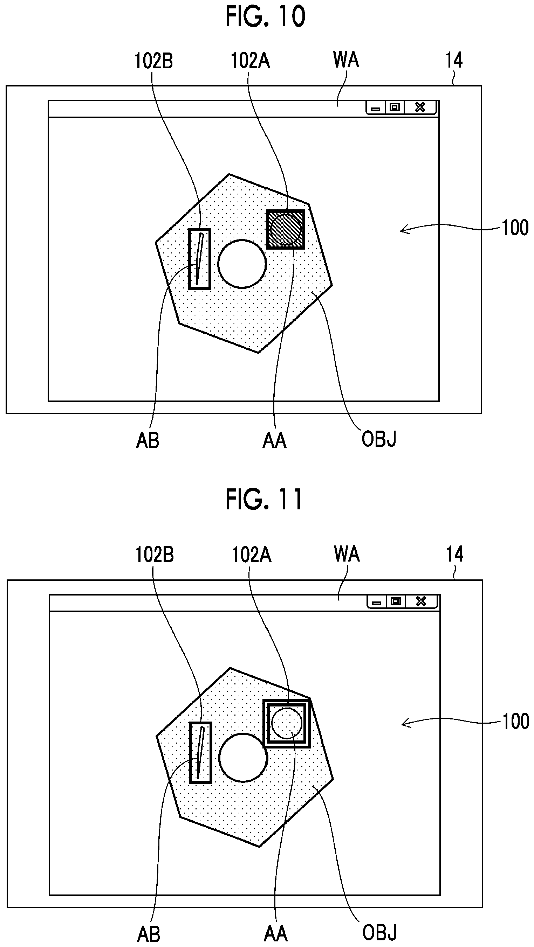

Fig. 10 is a diagram illustrating an example of the screen of the display 14 on which a final determination mark is superimposed and displayed. In an example illustrated in Fig. 10, an inside of the marker 102A is filled, as the final determination mark. By displaying in this way, the user can recognize a region where the final determination is made.

In addition, the user may be configured to enable remand of the interpretation. For example, the processor 16A receives an input of the region AA of the inspection target image 100 that needs to be redetermined, and superimposes and displays a mark indicating the remand on the input region AA. The user can input the redetermination using the input device 18.

Fig. 11 is a diagram illustrating an example of the screen of the display 14 on which a mark indicating remand is superimposed and displayed. In the example illustrated in Fig. 11, a bounding box of the marker 102A is displayed in double as the mark indicating the remand. By displaying in this way, the user can recognize the region to be reinterpreted.

Here, although the final determination mark and the mark indicating the remand are superimposed and displayed on the region AA, the processor 16A may give the final determination mark and the mark indicating the remand to the inspection target image 100 and store the image in the image database 20.

### [Information other than inspection target image]

The document information and the statistical information displayed on the screen of the display 14 are not limited to the first information which is the document information of the inspection target image 100 and the second information which is the statistical information of the non-document information, and may be information of a past determined image other than inspection target image 100.

For example, the processor 16A acquires determined interpretation information of a determined image in which a second inspection object different from the inspection object OBJ, which is the first inspection object, is imaged, and in which determined interpretation information by the plurality of determiners is associated with an arbitrary region, and displays at least one of third information, which is document information of the determined interpretation information related to the interpretation information associated with the region AA of the selected marker 102A, or fourth information, which is the statistical information obtained by statistically processing the non-document information of the determined interpretation information on the screen of the display 14. As an example, the document information of the determined image is displayed in place of the document information 120B on the screen of the display 14 illustrated in Fig. 6.

The second inspection object may be the same standard product as the inspection object OBJ. In addition, the third information may include at least one of the same text as the document information of the interpretation information associated with the region AA of the selected marker 102A, or text indicating a determination result for the same defect.

By displaying in this way, the user can recognize the information of the defect of an inspection object other than the inspection object OBJ, and can use it as a reference for the determination of the defect of the inspection object OBJ.

### [Notification of abnormality in determination result]

The image display device 16 may be configured to notify of an abnormality in the determination result of the determiner using statistical information. For example, the processor 16A calculates the distribution of the determination results of the plurality of determiners for the inspection target image 100, and in a case in which the variation in the determination results with respect to the inspection target image 100 is larger than a first criterion, the processor 16A displays that the variation in the determination is larger than that of the first criterion with respect to the determination results of the plurality of the determiners of the inspection target image 100, on the screen of the display 14.

Here, the variation in the determination results includes the variation in the distribution in a case in which the determination result is the determination information of a non-defective product and a defective product and the determination information of a type of the defect. In addition, the variation in the determination result includes the variance and the deviation in a case in which the determination result is the numerical information of the defect.

A case in which the determination results of the plurality of determiners are larger than the first criterion does not mean that all of the plurality of determiners have their own problems, and indicates that the determination of the inspection target image is difficult enough to have divided opinion. Therefore, the display indicating that the determination is varied may be performed for each determination result of each determiner, or may be displayed once for the entire determination result.

In addition, in a case in which the determination result of a specific determiner for the inspection target image 100 is an outlier, it may be displayed that there is a possibility that the determination result of the specific determiner is the outlier on the screen of the display 14.

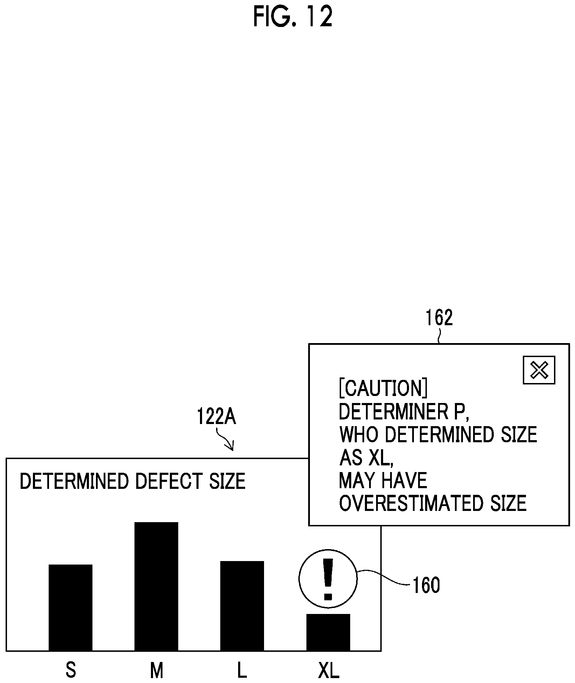

Fig. 12 is a diagram illustrating an example of statistical information 122A displayed on the screen of the display 14. The statistical information 122A is a histogram representing the distribution of the sizes of defects included in the region AA determined by the plurality of determiners. A horizontal axis indicates four types of sizes of small (S), medium (M), large (L), and extra large (XL) and a vertical axis indicates the number of determiners who have determined each size.

Here, it is assumed that only a determiner P determines the size as XL. In a case in which the processor 16A determines that the determination of the determiner P is the outlier, as illustrated in Fig. 12, the processor 16A displays a mark 160 indicating the outlier at a position of the XL size of the histogram. Furthermore, the processor 16A displays a caution display 162 indicating that the determination result of the determiner P overestimates the size of the defect. The mark 160 and the caution display 162 may be any display as long as it is recognizable by the user.

### [Weighting of determiners]

The statistical processing of the non-document information may be performed based on the determination record of each determiner. For example, the processor 16A performs statistical processing on the non-document information of the interpretation information by weighting the determination result of each determiner based on the determination records of each determiner of the plurality of determiners. The determination record of each determiner may be calculated using the statistical information of the non-document information of each of a plurality of determined images stored in the image database 20. The statistical information including the numerical information of the defect, the confidence level of the determination, and the measure of severity may be calculated by using weighted values according to the determination record of each determiner for the mean, the variance, and the deviation.

For the determiner who makes a determination in which the variation in the determination result with respect to the determined image is larger than that of the first criterion, and for a determiner who makes a determination in which the determination result is an outlier, the processor 16A makes the weight relatively small based on the determination record. On the other hand, for a determiner whose determination result is adopted as the final determination result at a high ratio, the processor 16A makes the weight relatively large based on the determination record. The processor 16A acquires the weighted statistical information in the statistical processing of the non-document information by using such weight for each determiner.

In addition, for the determination that the variation in the determination result with respect to the inspection target image 100 is larger than that of the first criterion, and the determination that the determination result of the specific determiner for the inspection target image 100 is an outlier, which have been described with reference to Fig. 12, the processor 16A may assume the determination results of a plurality of determined images stored in the image database 20 as a normal distribution, and may make a determination based on the distribution.

### [Others]

Here, although the image display device 16 used in the defect inspection system of non-destructive inspection has been described, the image display device 16 is not limited to the non-destructive inspection, and can be applied to display images for inspection and checking such as various inspection tasks for social infrastructure and the like.

The technical scope of the present invention is not limited to the scope described in the above-mentioned embodiment. The configurations and the like in each embodiment can be appropriately combined between the respective embodiments without departing from the gist of the present invention.

### Explanation of References

10: defect inspection system
12: imaging system
14: display
16: image display device
16A: processor
16B: memory
18: input device
20: image database
22: imaging room
24: imaging control unit
26: imaging operating unit
28: image recording unit
30: radiation detector
34: radiation source
100: inspection target image
102A: marker
102B: marker
104: cursor
106: range
110: enlarged inspection target image
120A: document information
120B: document information
120C: document information
122A: statistical information
130: scroll bar
132: slider
140: highlighted inspection target image
150: document creation image
160: mark
162: caution display
AA: region
AB: region
NW: network
OBJ: inspection object
S1 to S5: each step of an image display method
WA, WB, WC: window

## Claims

1. An image display device comprising:
at least one processor; and
at least one memory that stores an instruction to be executed by the at least one processor,
wherein the at least one processor
acquires a first inspection target image in which a first inspection object is imaged and in which interpretation information by a plurality of determiners is associated with an arbitrary region,
displays the first inspection target image on a display and displays a marker in at least one region with which the interpretation information of the first inspection target image is associated,
receives a selection instruction of the marker by a user,
displays a second inspection target image subjected to at least one of enlargement processing or highlight processing for a region including at least a part of a region of the marker for which the selection instruction is received in the first inspection target image, on the display, and
further displays at least one of first information, which is document information of the interpretation information associated with the region of the marker for which the selection instruction is received, or second information, which is statistical information obtained by statistically processing non-document information of the interpretation information, on the display.

2. The image display device according to claim 1,
wherein the document information includes a plurality of comments input by the plurality of determiners, respectively, and
the at least one processor displays a list of the plurality of comments.

3. The image display device according to claim 1 or 2,
wherein the non-document information includes determination results each classified by the plurality of determiners and including at least one of presence or absence, a type, or a severity of a defect in the region of the marker, and
the statistical information obtained by statistically processing the non-document information includes a distribution of the number of persons for each determination result.

4. The image display device according to any one of claims 1 to 3,
wherein the non-document information includes numerical information of a defect determined by each of the plurality of determiners, and
the statistical information obtained by statistically processing the non-document information includes at least one of mean or a distribution of the numerical information of the defect.

5. The image display device according to claim 4,
wherein the numerical information of the defect includes at least one of a size of the defect or a density of the defect.

6. The image display device according to any one of claims 1 to 5,
wherein the first inspection target image includes information of image processing in a case in which a determination, which is a basis of the interpretation information, is made, and
the at least one processor
receives a selection instruction by the user of at least one of the first information or the second information displayed on the display, and
displays the first inspection target image subjected to the image processing in a case in which a determination, which is a basis of the first information or second information for which the selection instruction is received, is made, on the display.

7. The image display device according to claim 6,
wherein the at least one processor
displays the first inspection target image subjected to the image processing together with the second inspection target image on the display.

8. The image display device according to claim 6,
wherein the at least one processor
displays the first inspection target image subjected to the image processing on the display in place of the second inspection target image.

9. The image display device according to any one of claims 6 to 8,
wherein the image processing includes at least one of reproduction of an enlargement ratio of an image, reproduction of a display position of the image, or a parameter of an appearance of the image, and
the parameter of the appearance of the image includes at least one of lightness and darkness of the image, sharpness of the image, contrast of the image, or gradation of the image.

10. The image display device according to any one of claims 1 to 9,
wherein the at least one processor
receives at least one of correction or approval of the interpretation information associated with the region, and
superimposes and displays a mark indicating a final determination on the region in which the at least one of correction or approval has been received.

11. The image display device according to any one of claims 1 to 10,
wherein the at least one processor
receives an input of a region of the first inspection target image that needs to be redetermined, and
superimposes and displays a mark indicating remand on the input region.

12. The image display device according to any one of claims 1 to 11,
wherein the at least one processor
acquires determined interpretation information of a determined image in which a second inspection object different from the first inspection object is imaged, and in which the determined interpretation information by the plurality of determiners is associated with an arbitrary region, and
displays at least one of third information, which is document information of the determined interpretation information related to the interpretation information associated with the region of the marker for which the selection instruction is received, or fourth information, which is statistical information obtained by statistically processing non-document information of the determined interpretation information on the display.

13. The image display device according to claim 12,
wherein the second inspection object is the same standard product as the first inspection object.

14. The image display device according to claim 12 or 13,
wherein the displayed document information of the determined interpretation information includes at least one of the same text as the document information of the interpretation information associated with the region of the marker for which the selection instruction is received, or text indicating a determination result for the same defect.

15. The image display device according to any one of claims 1 to 14,
wherein the at least one processor
calculates a distribution of determination results of the plurality of determiners for the first inspection target image, and
in a case in which a variation in the determination result with respect to the first inspection target image is larger than that of a first criterion, displays that the determination is varied with respect to the determination results of the plurality of determiners for the first inspection target image on the display.

16. The image display device according to any one of claims 1 to 15,
wherein the at least one processor
calculates a distribution of determination results of the plurality of determiners for the first inspection target image, and
in a case in which a determination result of a specific determiner with respect to the first inspection target image is an outlier, displays that there is a possibility that the determination result of the specific determiner is the outlier on the display.

17. The image display device according to claim 15 or 16,
wherein the at least one processor
performs the statistical processing on the non-document information of the interpretation information by weighting the determination result of each of the plurality of determiners based on a determination record of each of the determiners.

18. An image display method comprising:
an inspection target image acquisition step of acquiring a first inspection target image in which a first inspection object is imaged and in which interpretation information by a plurality of determiners is associated with an arbitrary region;
a first image display step of displaying the first inspection target image on a display and displaying a marker in at least one region with which the interpretation information of the first inspection target image is associated;
a reception step of receiving a selection instruction of the marker by a user;
a second image display step of displaying a second inspection target image subjected to at least one of enlargement processing or highlight processing for a region including at least a part of a region of the marker for which the selection instruction is received in the first inspection target image, on the display; and
an interpretation information display step of further displaying at least one of first information, which is document information of the interpretation information associated with the region of the marker for which the selection instruction is received, or second information, which is statistical information obtained by statistically processing non-document information of the interpretation information, on the display.

19. A program for causing a computer to execute the image display method according to claim 18.

20. A non-transitory computer-readable recording medium on which the program according to claim 19 is recorded.
